(19) Europäisches Patentamt / European Patent Office / Office européen des brevets

(11) **EP 2 807 270 B1**

(12) **EUROPEAN PATENT SPECIFICATION**

(45) Date of publication and mention
of the grant of the patent:
**06.04.2016 Bulletin 2016/14**

(51) Int Cl.:
*G01N 33/573* $^{(2006.01)}$    *C12Q 1/54* $^{(2006.01)}$

(21) Application number: **13701570.7**

(86) International application number:
**PCT/EP2013/000192**

(22) Date of filing: **23.01.2013**

(87) International publication number:
**WO 2013/110453 (01.08.2013 Gazette 2013/31)**

(54) **METHOD FOR THE EVALUATION OF THE QUALITY OF A TEST ELEMENT**

VERFAHREN ZUR AUSWERTUNG DER QUALITÄT EINES TESTELEMENTES

PROCÉDÉ POUR L'ÉVALUATION DE LA QUALITÉ D'UN ÉLÉMENT DE TEST

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.01.2012 EP 12000479**

(43) Date of publication of application:
**03.12.2014 Bulletin 2014/49**

(73) Proprietors:
• **Roche Diagnostics GmbH
68305 Mannheim (DE)**
Designated Contracting States:
**DE**
• **F.Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **HAAR, Hans-Peter
69168 Wiesloch (DE)**
• **HOENES, Joachim
64673 Zwingenberg (DE)**

• **HORN, Carina
68647 Biblis (DE)**
• **PETRICH, Wolfgang
76669 Bad Schönborn (DE)**
• **FREIFRAU VON KETTELER, Alexa
89075 Ulm (DE)**

(74) Representative: **Poredda, Andreas et al
Roche Diagnostics GmbH,
Patentabteilung,
Sandhofer Strasse 116
68305 Mannheim (DE)**

(56) References cited:
**EP-A2- 0 293 732    WO-A1-2009/015870
WO-A2-2010/105850    US-A- 6 055 060
US-A1- 2010 227 348    US-B2- 7 857 760**

• **EVANS N D ET AL: "Glucose-dependent changes
in NAD(P)H-related fluorescence lifetime of
adipocytes and fibroblasts in vitro: Potential for
non-invasive glucose sensing in diabetes
mellitus", JOURNAL OF PHOTOCHEMISTRY
AND PHOTOBIOLOGY B: BIOLOGY, ELSEVIER
SCIENCE S.A., BASEL, CH, vol. 80, no. 2, 1 August
2005 (2005-08-01) , pages 122-129, XP027789524,
ISSN: 1011-1344 [retrieved on 2005-08-01]**

## Description

## TECHNICAL FIELD OF THE INVENTION

The present application relates to a method for evaluating the quality of an analysis element which is useful for determining the concentration of an analyte in a liquid sample, and more particularly to the assaying of medical samples such as blood and/or interstitial liquid, for analytes contained therein, such as glucose. Furthermore, the application relates to an analysis system for determining the concentration of an analyte in a liquid sample which implements such a method.

## BACKGROUND

In methods for determining the concentration of analyte based on a fluorescence measurement, a reagent system is used which reacts with the sample resulting in a change of the amount of a fluorophore. This change of quantity can in principle be both an increase by formation of a fluorophore and also a decrease by consumption of a fluorophore. The reaction system is selected so that the change of the quantity of the fluorophore is characteristic of the desired analytical concentration. The analytical method includes the measurement of a measurement variable which correlates with the quantity of the fluorophore. The concentration is determined on the basis of the resulting measurement values by means of an evaluation algorithm.

Methods of this type are generally known. Reference can be made to the following documents, for example EP 0 293 732 A2, US 2005/0214891 A1, and US 2006/0003397 A1. All of these publications describe a similar test protocol, in which the reagent system contains the enzyme-coenzyme pair glucose dehydrogenase (GlucDH)/nicotinamide-adenine dinucleotide (NAD). Upon the reaction with this reagent system, under the influence of the GlucDH, a hydride ion is cleaved from the glucose and transferred to the NAD, so that NADH forms. The resulting quantity of NADH is characteristic of the glucose concentration. NADH is a strong fluorophore, whose concentration can be determined by a measurement of the fluorescence intensity. The correlation of the measured fluorescence intensity to the analyte concentration is typically determined by calibration. NADH is also a dye. Therefore, it can be favorable to additionally perform a photometric measurement (for example, measurement of the optical absorption at a specific wavelength).

This type of method has basically been known for some time. For example, reference is made in US 2005/0214891 A1 to a publication of Narayanaswamy et al. from the year 1988, in which a fluorescence measurement using GlucDH and NAD was employed for glucose determination. However, an array of fundamental problems is connected to the measurement of the fluorescence intensity, which are discussed, for example, in the book by J. R. Lakovicz, "Principles of Fluorescence Spectroscopy" (Springer Science and Business Media, 2006), and include the following:

o The intensity of the detected fluorescence signal is a function of a plurality of instrument-specific factors, such as the power of the light source, the transmission of the optical components in the light path, or the sensitivity of the detector. Uncontrolled changes of these factors impair the measurement precision.
○ A further error source in intensity measurements results from nonspecific light, which reaches the detector from the environment and can cause an uncontrolled signal change.
○ The intensity of the measured fluorescence light is not only a function of the quantity of the fluorophore. Rather it is also significantly influenced by its molecular environment in the sample. In particular processes which are summarized under the term fluorescence quenching contribute thereto in.
○ The position and orientation of the molecule can change between absorption and emission because in the statistical mean a time in the order of nanoseconds passes between the excitation of a molecule and the emission of a light quantum. Interfering influences result therefrom in regard to the fluorescence intensity, in particular temperature dependence.
○ The fluorescence is generally excited by ultraviolet light. Photochemical reactions of the electronically excited state may cause bleaching of the fluorophore. This is a further error source.

These problems may be reduced by calibration of the measurement of the fluorescence intensity using a fluorophore whose fluorescence intensity is known. However, this is very complex and is only suitable for sophisticated laboratory measurements.

WO 94/00602 A proposes an in vivo method for detecting an analyte in an individual, using a sensor comprising a fluorescent reagent for detecting said analyte, wherein the fluorescence may be measured e.g. by measuring changes in the excited state lifetime of said fluorophore.

EP 2 022 859 A proposes an improved measurement method wherein fluorescence lifetime measurements of a fluorophore are used in the determination of analyte concentration. Said improved method takes into account the afore-mentioned measurement errors and interferences, and is suitable to provide more reliable test results.

However, the state of the art in this context does not address the aspect of detecting and/or correcting irregular test results which arise from a degradation of the reagent test system used. Such degradation may occur in particular due to the effects of long storage times, including detrimental environmental conditions, such as high temperatures or high humidity. In these cases, the performance of the reagent test system used may be affected, indicating a result of an analyte level which is too low. This can be particularly critical because a test result may be displayed to the user without the error resulting from the poor performance of the reagent test system being detected.

On this basis, it is an object of the present invention to propose a method which allows with little expense an improved measurement precision, in particular in regard to the described degradation of the reagent test system. Moreover, the method should be suitable for small devices which operate as simply and cost-effectively as possible.

## SUMMARY

This object and others that will be appreciated by a person of ordinary skill in the art have been achieved according to the embodiments of the present invention disclosed herein.

In a first aspect, the present invention comprises a method for evaluating the quality of an analysis element which is useful for determining the concentration of an analyte in a liquid sample, comprising:

(a) providing an analysis element in which a reagent system is operatively integrated, wherein the reagent system contains at least one enzyme and at least one co-enzyme, wherein a reaction of the sample with the reagent system includes a complex formation of at least the enzyme and the co-enzyme, and

wherein a chemically modified form of the co-enzyme is a fluorophore;

(b) reacting the sample with the reagent system in order to produce a change of the quantity and/or of the luminescence properties of the fluorophore, wherein the quantity and/or the luminescence properties of the fluorophore relate to the concentration of the analyte;

(c) optionally measuring a first measurement variable,

(c') measuring at least a second measurement variable,

wherein the first and the second measurement variable are characteristic for the quantity of the fluorophore and/or for the degree of complexation of the fluorophore, and wherein the second measurement variable is the fluorescence lifetime of the fluorophore;

(d) optionally determining a first concentration $c1_{initial}$ of the fluorophore and/or of the analyte on the basis of the first measurement variable, using a pre-set enzyme activity $A_{Enz}$(preset);

(e) determining a second concentration $c2_{ref}$ of the fluorophore and/or of the analyte on the basis of the second measurement variable, using the pre-set enzyme activity $A_{Enz}$(preset); and/or determining the measured enzyme activity $A_{Enz}$(measured) on the basis of the second measurement variable and taking into account either the first concentration $c1_{initial}$ from step (d) or a known concentration $c1_{known}$ of the analyte in the sample; and

(f) evaluating the quality of the analysis element using an evaluation algorithm, wherein the evaluation algorithm comprises obtaining information relating to the measured enzyme activity $A_{Enz}$(measured), wherein said information is at least partly derived from the measurement of the fluorescence lifetime of the fluorophore, either directly by determining the measured enzyme activity $A_{Enz}$(measured) according to step (e), or indirectly by comparing the second concentration $c2_{ref}$ to the first concentration $c1_{initial}$.

It has been established in EP 2 022 859 A that in analysis systems in which the reagents of the reagent system are integrated in dry form in an analysis element, the fluorescence lifetime is surprisingly dependent to such a high degree on the analyte concentration that measurement values of this measurement variable may advantageously be used in the algorithm for determining the analytical result (desired concentration). This may be explained as follows for the case of a glucose test using the reagent system GlucDH/NAD.

The NADH formed as a result of the reaction of the glucose with the enzyme-coenzyme pair GlucDH/NAD forms a complex with the GlucDH. This complex formation influences the fluorescence lifetime of the NADH: the fluorescence of the free NADH is short-lived, because molecules in the surrounding liquid act as a quencher. In the complex, the NADH molecule is largely protected from the effect of quenchers and the fluorescence is significantly longer-lived.

These facts are known from literature. At the time of filing EP 2 022 859 A, however, it had been completely unexpected that this effect is so pronounced under the conditions prevailing in practical tests that the changes of the analyte concentration in blood samples occurring in practice result in a well-measurable shift of the mean fluorescence lifetime. On the basis of statements in the literature, it would have been expected that the degree of binding of the complex NADH/GlucDH is very high. The fluorescent light emitted by the complex NADH/GlucDH has a very much higher intensity than the fluorescence of free NADH. Therefore, it would be expected that the observed fluorescence lifetime depends only minimally from the free NADH and the lifetime effect caused by concentration changes in the physiological range is immeasurably low. However, it was established in the context of EP 2 022 859 A that under practical conditions a very high proportion of the NADH (over 90%) is not complexed.

According to the invention, optionally a first and at least a second measurement variable which are characteristic for the quantity of the fluorophore and/or for the degree of complexation of the fluorophore are measured, wherein the second measurement variable is the fluorescence lifetime of the fluorophore. Herein, the term "degree of complexation" refers to the amount of fluorophore being attached to the enzyme in the enzyme/co-enzyme complex (i.e. being complexed), as opposed to the fluorophore being present in its free form (i.e. without being attached to the enzyme).

The fluorescence lifetime provides a second, independent source of information about the fluorescence of the reaction products, in addition to the fluorescence intensity. It can be used independently (i.e., without other information) for determining the desired analyte concentration. However, it is preferably used in combination with the measurement of the fluorescence intensity. This does not require any additional expense for measurement technology, because typical methods for measuring the fluorescence lifetime simultaneously provide measurement results about the fluorescence intensity.

In order to use the fluorescence lifetime for the determination of the desired analyte concentration, it can be expedient to measure it and to introduce the measurement values resulting from the measurement into the evaluation algorithm in any suitable way. However, such a measurement (in the strict meaning, i.e., measurement of previously unknown measurement values) is not mandatory. Rather, the fluorescence lifetime can also be used in a way in which no measurement in the meaning that (previously unknown) measurement values of the fluorescence lifetime are determined, is required. One possibility is to measure the fluorescence intensity within a defined time window, the time window being adapted to the (known) fluorescence lifetime of a fluorophore resulting from the reaction of the sample with the reagent system. The measurement value resulting from such a measurement is related to the fluorescence lifetime. Hereafter it is also designated "lifetime-related fluorescence intensity". It may be used in the determining of the second concentration $c2_{ref}$ of the fluorophore and/or of the analyte on the basis of the second measurement variable. Therefore also in this case, it is correct to say that the fluorescence lifetime is used in the algorithm for determining the analyte concentration.

Due to the fact that a measurement of the fluorescence lifetime (in the explained strict meaning) is not absolutely necessary, the analysis instrument of a corresponding analysis system does not necessarily have to have a measuring unit which is adapted for measuring the fluorescence lifetime of the fluorophore. Rather, the possibility alternatively or additionally exists that the measuring unit of the analysis instrument is implemented so that it considers the fluorescence lifetime in another way, for example, using the discussed time window which is set during the intensity measurement.

Suitable methods for measuring the fluorescence lifetime are known. One means that is, in principle, suitable for the invention is a time-resolved fluorescence detection, in which a very short pulse of excitation light is emitted and the curve of the resulting emission curve is detected using suitable detection methods having high time resolution. However, a phase modulation method operating with continuous emission is preferably used in the context of the invention. More detailed information about suitable methods may be taken from the literature. Reference is to be made in particular to the book of Lakowicz cited above, and to the following further publications, as well as the literature cited therein: T. G. Scott et al., "Emission Properties of NADH. Studies of Fluorescence Lifetimes ... " (J. Am. Chem. Soc., 1970,687-695); U.S. Pat. No. 5,485,530; and EP 0 561 653 A1.

According to the invention, a chemically modified form of the co-enzyme is a fluorophore. Thus, any co-enzyme may be used in the invention which can be transformed into a modified form having fluorescence properties. Co-enzymes which can be transformed into a fluorophore by a rather simple chemical change are particularly useful for the purpose of the invention: This is the case e.g. if the chemical change is an oxidation or a reduction, which may be effected by simple transformation steps such as protonation, de-protonation, or hydride transfer, which transformation steps may advantageously be promoted by enzymatic action. Preferably, the co-enzyme is chemically modified into a fluorophore during reaction of the sample with the reagent system. When NAD or derivatives thereof, such as carbaNAD, carbaNADP and derivatives thereof, are used in the invention, then usually NAD (or the corresponding derivatives thereof, respectively) acts as the co-enzyme, whereas the corresponding reduced form (such as NADH, carbaNADH, etc., i.e. the chemically modified form of the co-enzyme) represents the fluorophore. In the context of the present invention, since the fluorophore is a modified form of the co-enzyme, the term "co-enzyme" may also sometimes be employed when reference is made to the fluorophore (despite the fact that the fluorophore may itself not act as a co-enzyme, as it is the case e.g. for the pairs of co-enzyme and fluorophore comprising NAD/NADH, carbaNAD/carbaNADH, and their corresponding derivatives).

Optionally, the method of the invention makes use of the pre-set enzyme activity $A_{Enz}$(preset), namely in the determining of the first concentration $c1_{initial}$ of the fluorophore and/or of the analyte on the basis of the first measurement variable, and/or in the determining of the second concentration $c2_{ref}$ of the fluorophore and/or of the analyte on the basis of the second measurement variable. It is to be noted that said pre-set enzyme activity $A_{Enz}$(preset) does not need to be actually measured for carrying out the method of the invention. Rather, it may be sufficient to ensure that there is an amount of enzyme present in the reagent system which provides for an overall enzyme activity which is, under usual measurement conditions, large enough such that the reaction of the sample with the reagent system will proceed nearly until a reaction equilibrium is reached. For example, if in the determining of the first concentration $c1_{initial}$, the first measurement variable is absorption of the fluorophore, such as by using a reflection measurement, then the actual specific value of $A_{Enz}(preset)$ is not needed, but $A_{Enz}(preset)$ is implicitly considered (namely, by assuming that $A_{Enz}(preset)$ is sufficiently high such that $c1_{initial}$ can be reasonably determined). Typically, the pre-set enzyme activity $A_{Enz}(preset)$, or at least its order of magnitude, can be derived or estimated from the amount of enzyme as employed in the reagent test system.

The evaluation algorithm may comprise determining if the enzyme activity $A_{Enz}(measured)$ is below a predetermined threshold T1. Said threshold T1 e.g. may be an absolute minimum value of enzyme activity $A_{Enz}(min)$ which is known to be necessary in order to carry out a reliable analyte measurement using the reagent system of the invention. As another advantageous option, which may be used additionally or alternatively, the evaluation algorithm may comprise determining if the difference between the enzyme activity $A_{Enz}(measured)$ and the pre-set enzyme activity $A_{Enz}(preset)$ is above a predetermined threshold T2.

Additionally or alternatively, the evaluation algorithm comprises determining if the difference between the first concentration $c1_{initial}$ from step (d) and the second concentration $c2_{ref}$ from step (e) is above a predetermined threshold T3.

In one embodiment of the present invention, the analysis element is rejected if the enzyme activity $A_{Enz}(measured)$ is below the predetermined threshold T1. Preferably, the analysis element is rejected if the difference, i.e. the absolute value thereof, between the enzyme activity $A_{Enz}(measured)$ and the pre-set enzyme activity $A_{Enz}(preset)$ is above the predetermined threshold T2. Additionally or alternatively, it is as well preferred that the analysis element is rejected if the difference, i.e. the absolute value thereof, between the first concentration $c1_{initial}$ from step (d) and the second concentration $c2_{ref}$ from step (e) is above the predetermined threshold T3.

In this context, it is worth noting that the method of the invention may advantageously be employed by using a control solution (i.e. the sample) of known analyte concentration $c1_{known}$, e.g. a solution containing a known amount of glucose, thus avoiding any need to determine the first and the second concentration $c1_{initial}$ and $c2_{ref}$, respectively. According to step (e), the measured enzyme activity $A_{Enz}(measured)$ may be determined on the basis of the second measurement variable (i.e. from the fluorescence lifetime measurement) and taking into account the known concentration $c1_{known}$ of the analyte in the control solution. The measured enzyme activity $A_{Enz}(measured)$ may then be used to evaluate the quality of the test element as described herein before. As a result, it may be concluded that one or more test elements, e.g. the test strips from an open package or container, should not be used any more.

In another preferred embodiment of the present invention, the information about the enzyme activity $A_{Enz}(measured)$ is used to determine a corrected first concentration $c1_{corr}$ of the fluorophore and/or of the analyte.

Thus, according to a preferred embodiment, the first concentration $c1_{initial}$ of the fluorophore and/or of the analyte is determined according to step (d) on the basis of the first measurement variable, using a pre-set enzyme activity $A_{Enz}(preset)$, and the measured enzyme activity $A_{Enz}(measured)$ is determined according to step (e) on the basis of the second measurement variable and using the first concentration $c1_{initial}$ from step (d). Then it is determined if $A_{Enz}(measured)$ is below a predetermined threshold T1, and/or if the difference between $A_{Enz}(measured)$ and the pre-set enzyme activity $A_{Enz}(preset)$ is above a predetermined threshold T2. If one or both of these conditions are fulfilled, then it can be concluded that the enzyme activity actually present in the reagent test system (corresponding to $A_{Enz}(measured)$) differs from the pre-set enzyme activity $A_{Enz}(preset)$ to such an extent that either the test element should be rejected, or that a corrected first concentration $c1_{corr}$ of the fluorophore and/or of the analyte should be calculated, e.g. on the basis of a different calibration curve for the reagent system, which calibration curve is specifically related to or adapted to a lower enzyme activity.

In a more preferred embodiment, the determining of the corrected first concentration $c1_{corr}$ comprises an iterative algorithm.

Typically, the first measurement variable may be one of absorption and fluorescence intensity, preferably absorption. In the context of the present invention, the term "absorption" shall comprise any measurement arrangement which is suitable to detect at least some degree of optical absorption. In particular, the optical absorption may be measured by reflection (also referred to as remission) and/or transmission measurements.

In another embodiment, the first measurement variable is absorption, and the method further comprises measuring a third measurement variable which is characteristic for the quantity of the fluorophore, wherein the third measurement variable is fluorescence intensity, and wherein the first, the second and the third measurement variable are used in the evaluation algorithm.

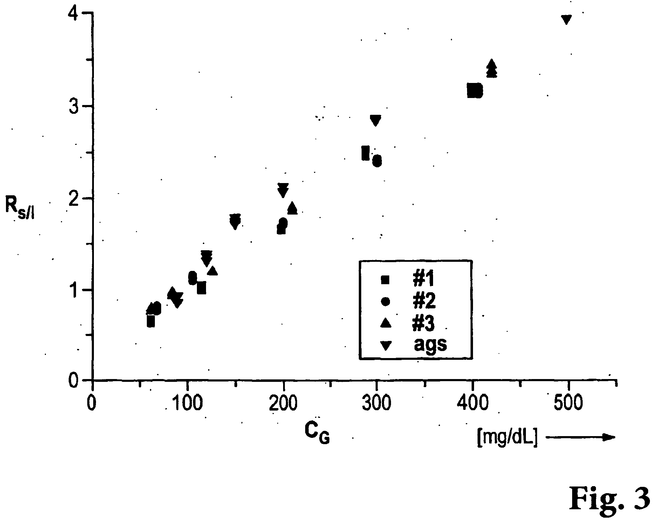

Advantageously, the measurement of the fluorescence lifetime is used to determine a lifetime-related value of the fluorescence intensity (LRFI). The measurement of LRFI values is an example of the fact that in the context of the invention a measurement of a fluorescence lifetime (in the strict meaning) is not absolutely necessary. Rather, by using LRFI values, the measurement of the fluorescence lifetime can be efficiently effected via measurements of fluorescence intensities, wherein the measurements of fluorescence intensity are carried out in a specifically adapted time frame. This will be elucidated below in more detail with regard to FIG. 3.

In a preferred embodiment, the fluorescence intensity is measured for at least two different fluorescence lifetimes in order to determine in each case lifetime-related values of the fluorescence intensity.

In a more preferred embodiment, the two lifetime-related values of the fluorescence intensity are related to one another.

Additionally, the measured fluorescence lifetime may be used in a compensation algorithm to compensate for measurement errors which are caused by interfering variables, such as the temperature or humidity conditions, particularly at the time of measurement, or the hematocrit level of the sample.

The fluorophore is preferably selected from the group comprising NADH and NADPH, including their derivatives. In particular, derivatives are suitable in which the molecule part essentially responsible for the fluorescence remains unchanged. In such derivatives, basically similar fluorescence properties are to be expected. Of course, the parameters of the fluorescence, such as the wavelength of the absorption or the emission, may change. Due to the fact that the pyridine ring is essentially responsible for the fluorescence in NAD/NADH, derivatives in which the pyridine ring is not modified are particularly suitable. Derivatives suitable for the present invention are described, for example, in WO 2007/012494 and US 2007/0026476. CarbaNAD, a derivative without glycosyl binding which was already described in 1988, is particularly suitable for use in the method according to the invention (J. T. Slama, Biochemistry 1989, 27, 183 and Biochemistry 1989, 28, 7688). The ribose is substituted therein by a carbocyclic sugar unit. Of course, not all derivatives of NADH and NADPH are equally suitable for the invention. The suitability for use according to the invention can, however, be experimentally examined without problems. Preferably, the fluorophore is NADH or NADPH or a derivative thereof, whose pyridine ring is not modified, in particular carbaNADH or carbaNADPH or a derivative thereof.

Typically, the enzyme may be selected from the group comprising glucose dehydrogenase (E.C.1.1.1.47), lactate dehydrogenase (E.C.1.1.1.27, 1.1.1.28), malate dehydrogenase (E.C.1.1.1.37), glycerine dehydrogenase (E.C.1.1.1.6), alcohol dehydrogenase (E.C.1.1.1.1), amino acid dehydrogenase, such as L-amino acid dehydrogenase (E.C.1.4.1.5), alpha-hydroxybutyrate dehydrogenase, sorbitol dehydrogenase, glucose oxidase (E.C.1.1.3.4), cholesterol oxidase (E.C.1.1.3.6), amino transferases, such as aspartate or alanine amino transferase, 5'-nucleotidase, creatin kinase, glucose 6-phosphate dehydrogenase (E.C.1.1.1.49), NAD dependent cholesterol dehydrogenase (E.C.1.1.1.62), and FAD dependent glucose dehydrogenase (E.C. 1.1.99.10). Preferably, the enzyme is selected from the group consisting of glucose dehydrogenase (E.C.1.1.1.47), alcohol dehydrogenase (E.C.1.1.1.1), and glucose 6-phosphate dehydrogenase (E.C.1.1.1.49); more preferably, the enzyme is one of glucose dehydrogenase (E.C.1.1.1.47) and glucose 6-phosphate dehydrogenase (E.C.1.1.1.49). Most preferably, the enzyme is glucose dehydrogenase (E.C.1.1.1.47).

Typically, the liquid sample to be analyzed may be a bodily fluid, specifically blood or interstitial fluid. Preferably, the analyte is glucose.

The most prominent advantage of the present invention is that a degradation of the reagent test system can be detected and/or can be corrected for. Inter alia, the following additional advantages may be achieved by the present invention:

  ∘ The problems discussed above are largely avoided. In particular, a calibration of the fluorescence intensity, using a standard fluorophore, is not necessary.
  ∘ The high-frequency measurement results in a reduction of the influence of interfering ambient light.
  ∘ The invention allows increased precision, in particular by elimination or reduction of error sources.

The invention is to be explained in more detail by the following figures and examples.

## BRIEF DESCRIPTION OF THE DRAWINGS

The following detailed description of the embodiments of the present invention can be best understood when read in conjunction with the following drawings, where like structure is indicated with like reference numerals and in which:

  FIG. 1 shows a schematic sketch of the functional components of an analysis system suitable for embodiments of the present invention.
  FIG. 2 shows measurement results in regard to the mean lifetime of the fluorescence as a function of the ratio of the concentrations of GlucDH and NADH for various temperatures.
  FIG. 3 shows a measurement result in regard to the functional relationship between the glucose concentration and the intensity ratio between short-lived and long-lived fluorescence.
  FIG. 4 shows a measurement result of the mean fluorescence lifetime versus glucose concentration measured on test strips.
  FIG. 5 shows a measurement result of the mean fluorescence lifetime versus enzyme activity.
  FIG. 6 shows an example of a preferred embodiment of the present invention schematically illustrating an iterative evaluation algorithm for correcting the initially determined concentration $c1_{initial}$ by taking into account information about the enzyme activity $A_{Enz}$.

In order that the present invention may be more readily understood, reference is made to the following detailed descriptions and examples, which are intended to illustrate the present invention, but not limit the scope thereof.

## DETAILED DESCRIPTION OF EMBODIMENTS OF THE PRESENT INVENTION

The following descriptions of the embodiments are merely exemplary in nature and are in no way intended to limit the present invention or its application or uses.

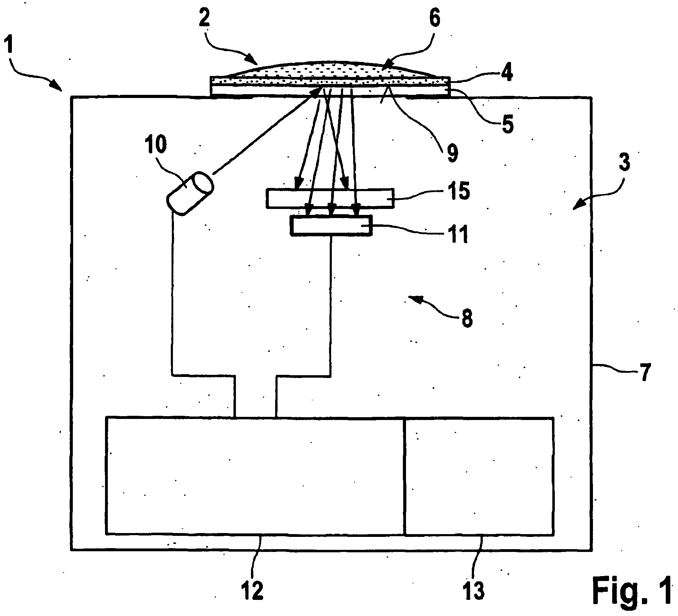

The analysis system shown in very schematic form in FIG. 1 comprises two components adapted to one another, namely an analysis element **2** and an analysis instrument **3**. The analysis element **2** comprises a reagent layer **4,** which contains a reagent system in a suitable matrix, such as a gel matrix. The reagent layer **4** is located on an optically transparent carrier **5**. The reagents of the reaction system are embedded in the matrix of the reagent layer **4** in dry form. When a sample liquid **6** is dispensed onto the free surface of the reagent layer **4** and penetrates therein, the reagents of the reagent system are dissolved and a reaction of the sample liquid occurs, which results in a change of the quantity of a fluorophore, which is a component of the reagent system. Further information about such analysis elements is provided, for example, in the documents U.S. Pat. No. 3,802,842; U.S. Pat. No. 4,061,468; and US 2006/0003397.

The housing **7** of the analysis instrument **3** contains a measuring unit, designated identified as a whole by **8**. It is adapted for determining a measurement variable which is characteristic for the quantity of the fluorophore. The measuring unit **8** includes an excitation light source **10,** such as a laser or an LED, whose light is directed from the back side (through the optically transparent carrier **5**) onto a surface of the reagent layer **4** which faces away from the sample dispensing side and toward the optically transparent carrier **5**. Secondary light is emitted from the analysis zone **9** toward a detector **11** and is detected thereby. The resulting measuring signal is supplied to a signal processing unit **12** and amplified, processed, and digitized in a usual manner. The resulting digitized measurement values are supplied to an analysis unit **13** which contains the required software and hardware to determine the desired concentration of the analyte from the digitized measurement values.

The measuring unit **8** is adapted for detecting fluorescent light emitted from the analysis zone **9**. For this purpose, the access of the primary light originating from the excitation light source **10** is blocked, using typical means such as a filter element **15** shown in FIG. 1, in such a manner that the fluorescent light, which is emitted at a longer wavelength, can be detected selectively by the detector **11.**

So far the design of the analysis system according to the invention is conventionally and therefore does not have to be explained in greater detail. More detailed information is available, for example, from the cited documents of the prior art.

A special feature of the analysis system according to the invention shown in FIG. 1 is that the measuring unit **8** is capable of measuring the fluorescence lifetime and/or to take it into account (in the context of the measurement of another measurement variable). As already noted, the fluorescence lifetime measurement is preferably performed using a phase modulation method. The light emitted from the excitation light source **10** is modulated at high frequency and the desired information about the fluorescence lifetime is determined from a phase shift which is measured by means of the detector **11** and the signal processing unit **12.** Again a more detailed explanation in this regard is not necessary, because supplementary information is available from the literature, in particular the above-mentioned documents.

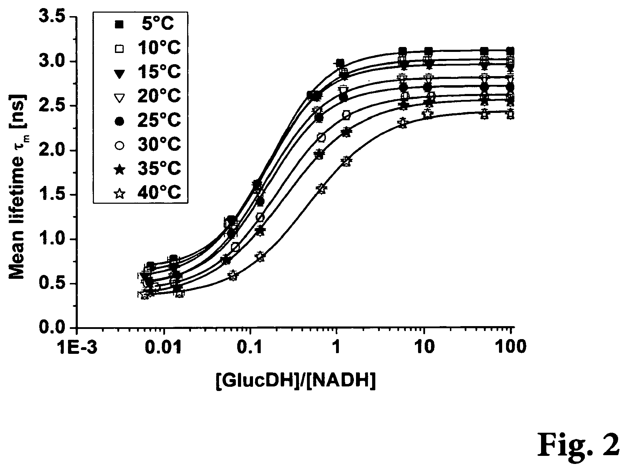

FIG. 2 shows experimental results from the research work which contributed to the invention. The mean fluorescence lifetime $\tau_m$ is shown for various concentration ratios of a liquid mixture of GlucDH and NADH. A $23\mu M$ NADH solution having varying concentrations of GlucDH ($11.5\mu M$ to 2.3mM) was used to prepare the concentrations specified on the abscissa. The measurements were carried out keeping the optical density smaller than 0.2 and at different temperatures in the range of 5°C to 40°C. The fluorescence decay was then fitted using a three exponential model, whereby the two lifetimes of free NADH and the complexed lifetime were kept constant. In order to determine the lifetime of complexed NADH at different temperatures in the range of 5°C to 40°C, NAD was reduced to NADH by Glucose, catalyzed by GlucDH in a different experiment. The fluorescence decay was then fitted using a three exponential model, whereby the two lifetimes of free NADH were kept constant.

FIG. 2 shows that the shift of the ratio between free and complexed NADH, which is caused by the variation of the concentration of GlucDH, can be observed well by lifetime measurements, the measurement curve approaching the limiting values $\tau_1$=2.98 ns and $\tau_2$=0.42 ns for very high and very low GlucDH concentrations. The fluorescence lifetime is a measurement variable which is characteristic of the quantity of the fluorophore, as shown by the fact that the degree of complex formation is a function of the amount of the fluorescent species.

As illustrated in Fig. 2, the change in the mean fluorescence lifetime is largest for a concentration ratio of enzyme to coenzyme in-between 0.1 and 1. Therefore, it may be advantageous to adapt the concentration of GlucDH in future test elements in a way that the concentration ratio of enzyme to coenzyme is within this range.

Additionally, Fig. 2 shows that the mean fluorescence lifetime is also dependent on temperature. While the shape of the curve appears to stay constant, the mean lifetimes shift to higher values for lower temperatures. From a practical perspective, the mean lifetime may thus be used for a conceivable temperature correction of an analyte sensing system, such as e.g. a glucose monitoring test system.

In the context of the invention it was derived from the measurement results shown in FIG. 2 that the change of the lifetime can be used to get additional information for quantifying an analyte in fluorescence tests, in particular information relating to the enzyme activity as explained herein. These findings can be used analytically in various ways. For example, for the purpose of the invention and analogous to FIG. 2, the mean fluorescence lifetime $\tau_m$ may be plotted versus various ratios of the enzyme activity (e.g. of GlucDH) to NADH concentration.

FIG. 3 shows an example of a preferred way of measuring the second measurement variable in order to gain information therefrom for the determination of the first concentration $c2_{ref}$ and/or $A_{Enz}$(measured). Particularly, FIG. 3 illustrates a possibility in which the measurement of the fluorescence lifetime is used for determining a lifetime-related fluorescence intensity (LRFI). Such an LRFI value describes the intensity of the fluorescence as a function of the fluorescence lifetime and thus forms a measure of the relationship of the quantity of a plurality of fluorescent species, which are distinguished by different mean lifetimes, such as complexed NADH in proportion to free NADH. An LRFI value can be determined, for example, on the basis of fluorescence signals, which are excited by short light pulses and are measured at high resolution of time. The decay of the fluorescence signal is a logarithmic function, which is a function of the mean fluorescence lifetimes of the fluorescent species and their concentration. The separate intensities of the two signal components may be determined by mathematical fitting of such measurement curves.

FIG. 3 shows the dependence of such LRFI values on the glucose concentration for a glucose fluorescence test using analysis elements as are described in US 2005/0214891 A1 and US 2006/0003397 A1. A ratio $R_{s/l}$ between the LRFI value for short-lived free NADH and the LRFI value for long-lived complexed NADH is shown. The measurements were performed using blood samples of different hematocrit values and using an aqueous glucose solution. The resulting measurement values are identified by different symbols in FIG. 3. Symbols for #1 through #3 indicate the numbers of samples having different hematocrit concentrations (very high-normal range-very low); "ags" means "aqueous glucose solution". It was shown that the correlation between the glucose concentration and the $R_{s/l}$ value is very good, the measured values being largely independent of the hematocrit value, i.e., of the concentration of the blood cells in the sample.

In FIG. 3, two LRFI values are related to one another. $R_{s/l}$ was calculated according to

$$R_{s/l} = \frac{A_2 \cdot \tau_2}{A_1 \cdot \tau_1}$$

Therein A designates the amplitude, i.e., a measure of the intensity, and $\tau$ designates the fluorescence lifetime of the two fluorescent species having different lifetimes. The value $R_{s/l}$ is calibrated against the analyte concentration (or, in a first step, against the concentration of the fluorophore) and then used in an algorithm as a measure for the analyte (or the fluorophore) concentration.

There are numerous other possibilities for relating LRFI values to one another so that a computed variable is formed, which can be used, via a calibration, as a measure for the desired analyte (or fluorophore) concentration. For example, a mean fluorescence lifetime (referred to as $\tau_m$ here) can be calculated according to

$$\tau_m = \frac{A_1\tau_1 + A_2\tau_2}{A_1 + A_2}$$

It is particularly preferred to perform an area-weighted averaging according to

$$\tau_m = \frac{A_1\tau_1^2 + A_2\tau_2^2}{A_1\tau_1 + A_2\tau_2}$$

All of these examples share the feature that the fluorescence lifetimes and the fluorescence intensities of two fluorescent species are used as characteristic measurement variables for the analysis. They are related to one another according to a predefined mathematical equation in order to determine a computed variable, which can be calibrated against the desired analyte (or fluorophore) concentration. The use of two simultaneously measured values and the calculation of a quotient has the result that measurement errors which influence both fluorescence measurement values in the same way, for example, due to contaminants or small disturbances in the optical measuring channel, cause no or little deterioration of the measurement result, less than in previously known measurements of the fluorescence intensity.

The measurement of LRFI values is an example of the fact that in the context of the invention, a measurement of a fluorescence lifetime (in the strict meaning) is not absolutely necessary. As described above, the measuring unit of the analysis instrument can also be implemented so that it detects a fluorescence intensity of a predefined time window and an LRFI value is thus measured. Preferably two or more LRFI values of this type are related to one another in order to determine a computed variable which can be calibrated against the analyte concentration, similarly as explained in the above examples.

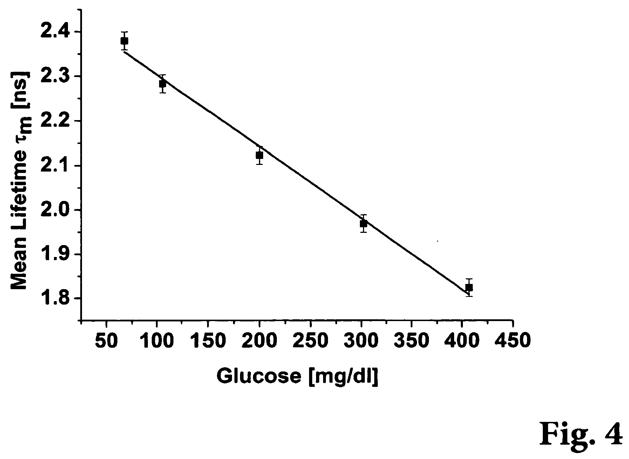

Figure 4 shows the mean fluorescence lifetime $\tau_m$ versus the glucose concentration, as measured on test strips using a confocal microscope (Axiovert 100, Zeiss, Germany) equipped with a water immersion objective (NA=1,2, Plan Apo, Olympus, Japan). The test strips were excited with a pulsed laser diode (LD) at 375nm. Blood with varying concentrations of glucose was applied on several test strips, containing NAD and GlucDH. The mean fluorescence lifetime $\tau_m$ exhibits a linear behavior with glucose concentration.

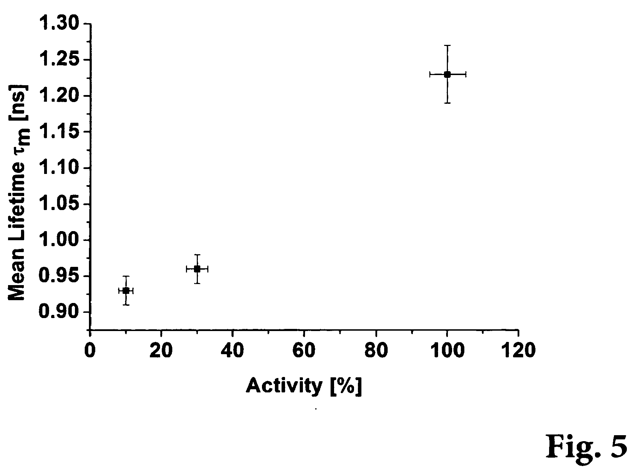

FIG. 5 shows the mean fluorescence lifetime $\tau_m$ versus enzyme activity. These experiments were carried out using fluorescence lifetime imaging measurements on glass substrates to which the reagents had been applied, wherein the fluorescence lifetime was monitored in two time frames, namely 0.5-1 ns, and 3-3.5 ns). The mean fluorescence lifetime $\tau_m$ decreases with enzyme activity. This can be explained as follows: The less active the enzyme is, the higher is the fraction of molecules of NADH which is present in their free form, i.e. which are not attached to the enzyme, such that the mean fluorescence lifetime $\tau_m$ will gradually point to the fluorescence lifetime of free NADH. In this way, the dependence of the fluorescence lifetime of the fluorophore from the (actual) enzyme activity could be shown.

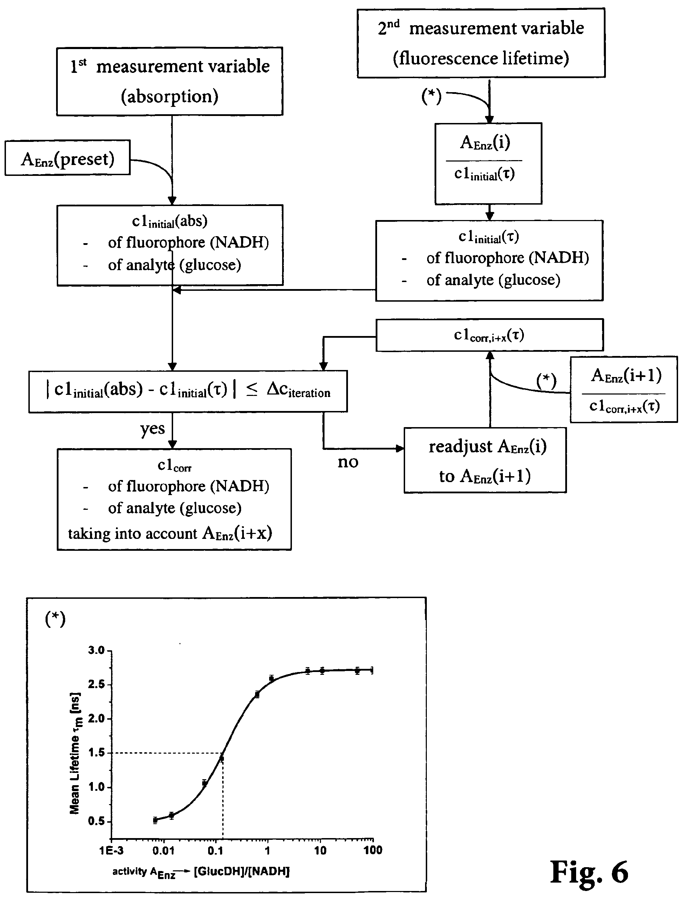

FIG. 6 shows an example of a preferred embodiment of the present invention schematically illustrating an iterative evaluation algorithm for correcting the initially determined concentration $c1_{initial}$ by taking into account information about the enzyme activity $A_{Enz}$. This example assumes a system wherein as a first measurement variable the absorption of the fluorophore NADH is measured, and as a second measurement variable the fluorescence lifetime of NADH is measured, according to step (c) and (c'), respectively. From the absorption measurement, a first concentration $c1_{initial}(abs)$ of NADH (or of the analyte, i.e. glucose) can be determined using the first measurement variable and a pre-set enzyme activity $A_{Enz}(preset)$. From the measured fluorescence lifetime of NADH, information is obtained relating to the ratio of an enzyme activity $A_{Enz}(i)$ (which e.g. may correspond to the enzyme activity $A_{Enz}(preset)$) to a concentration $c1_{initial}(\tau)$ of NADH. Since the concentration $c1_{initial}(\tau)$ of NADH depends on the two variables (a) measured fluorescence lifetime of NADH, and (b) the actual enzyme activity $A_{Enz}(actual)$, and since the fluorescence lifetime of NADH is known at this stage, $c1_{initial}(\tau)$ of NADH may be calculated from the measured fluorescence lifetime of NADH by using the enzyme activity $A_{Enz}(i)$ as an initial enzyme activity to begin an iteration process. Next, the difference between the first concentration $c1_{initial}(abs)$ of NADH from the absorption measurement is compared to the calculated concentration $c1_{initial}(\tau)$ of NADH from the fluorescence lifetime measurement. If said difference (i.e. the absolute value thereof) is above a pre-set threshold determining the iteration process (e.g. above 1 mg/dL), then the enzyme activity $A_{Enz}(i)$ should be adjusted to a more suitable value $A_{Enz}(i+1)$. Using the modified enzyme activity $A_{Enz}(i+1)$, a corrected concentration $c1_{corr}(\tau)$ may be calculated. The calculated corrected

concentration $c1_{corr}(\tau)$ of NADH (being based on the fluorescence lifetime measurement) may again be compared to the first concentration $c1_{initial}(abs)$ of NADH (being based on the absorption measurement). This iteration process may proceed until the iteration criterion is fulfilled, e.g. until the difference between $c1_{initial}(abs)$ of NADH and $c1_{corr,i+x}(\tau)$ of NADH is below a pre-set threshold, thereby obtaining an adjusted enzyme activity $A_{Enz}(i+x)$, with x being an integer. Taking the adjusted enzyme activity $A_{Enz}(i+x)$ into account, a corrected concentration $c1_{corr}(abs)$ of NADH, and hence a corrected concentration of the analyte of interest, may be calculated, e.g. by using a different calibration curve which is more appropriate for a lower enzyme activity, and may be displayed to the user.

In the context of the present invention, it is to be understood that the term "determining a concentration" may include the case where said concentration value itself actually is not calculated, but where the signal intensity obtained from the measurement is employed. As the skilled person will readily appreciate, this approach is feasible because the measured signal intensity is related to any concentration value derived therefrom. In particular, this applies to determining a first concentration $c1_{initial}$ of the fluorophore and/or of the analyte on the basis of the first measurement variable, and to determining a second concentration $c2_{ref}$ of the fluorophore and/or of the analyte on the basis of the second measurement variable.

For the purpose of the invention, not only the described system, in which glucose is determined with the aid of GlucDH and NAD, may be used. Rather, very generally cases are suitable in which a fluorophore is used which fluoresces in at least two species having different fluorescence lifetimes. In particular, according to the invention the reaction of the sample with the reagent system includes complex formation with participation of at least two molecules, namely an enzyme/coenzyme pair, wherein a chemically modified form of the co-enzyme is a fluorophore.

The features disclosed in the above description, the claims and the drawings may be important both individually and in any combination with one another for implementing the invention in its various embodiments.

It is noted that terms like "preferably", "commonly", and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

For the purposes of describing and defining the present invention it is noted that the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

Having described the present invention in detail and by reference to specific embodiments thereof, it will be apparent that modifications and variations are possible without departing from the scope defined in the claims. More specifically, although some aspects of the present invention are identified herein as preferred or particularly advantageous, it is contemplated that the invention is not necessarily limited to these preferred aspects.

**Claims**

1. A method for evaluating the quality of an analysis element which is useful for determining the concentration of an analyte in a liquid sample, comprising:

   (a) providing an analysis element in which a reagent system is operatively integrated, wherein the reagent system contains at least one enzyme and at least one co-enzyme, wherein a reaction of the sample with the reagent system includes a complex formation of at least the enzyme and the co-enzyme, and wherein a chemically modified form of the co-enzyme is a fluorophore;
   (b) reacting the sample with the reagent system in order to produce a change of the quantity and/or of the luminescence properties of the fluorophore, wherein the quantity and/or the luminescence properties of the fluorophore relate to the concentration of the analyte;
   (c) optionally measuring a first measurement variable;
   (c') measuring at least a second measurement variable,
   wherein the first and the second measurement variable are characteristic for the quantity of the fluorophore and/or for the degree of complexation of the fluorophore, and wherein the second measurement variable is the fluorescence lifetime of the fluorophore;
   (d) optionally determining a first concentration $c1_{initial}$ of the fluorophore and/or of the analyte on the basis of the first measurement variable, using a pre-set enzyme activity $A_{Enz}$(preset);
   (e) determining a second concentration $c2_{ref}$ of the fluorophore and/or of the analyte on the basis of the second measurement variable, using the pre-set enzyme activity $A_{Enz}$(preset); and/or determining the measured enzyme activity $A_{Enz}$(measured) on the basis of the second measurement variable and taking into account either the first concentration $c1_{initial}$ from step (d) or a known concentration $c1_{known}$ of the analyte in the sample; and
   (f) evaluating the quality of the analysis element using an evaluation algorithm, wherein the evaluation algorithm comprises obtaining information relating to the measured enzyme activity $A_{Enz}$(measured), wherein said information is at least partly derived from the measurement of the fluorescence lifetime of the fluorophore, either directly by determining the measured enzyme activity $A_{Enz}$(measured) according to step (e), or indirectly by comparing the second concentration $c2_{ref}$ to the first concentration $c1_{initial}$.

2. The method according to claim 1, wherein the evaluation algorithm comprises determining if the enzyme activity $A_{Enz}$(measured) is below a predetermined threshold T1, and/or determining if the difference between the enzyme activity $A_{Enz}$(measured) and the pre-set enzyme activity $A_{Enz}$(preset) is above a predetermined threshold T2.

3. The method according to claim 1 or 2, wherein the evaluation algorithm comprises determining if the difference between the first concentration $c1_{initial}$ from step (d) and the second concentration $c2_{ref}$ from step (e) is above a predetermined threshold T3.

4. The method according to claim 2 or 3, wherein the analysis element is rejected if the enzyme activity $A_{Enz}$(measured) is below the predetermined threshold T1, and/or if the difference between the enzyme activity $A_{Enz}$(measured) and the pre-set enzyme activity $A_{Enz}$(pre-set) is above the predetermined threshold T2, and/or if the difference between the first concentration $c1_{initial}$ from step (d) and the second concentration $c2_{ref}$ from step (e) is above the predetermined threshold T3.

5. The method according to any of the preceding claims, wherein the information about the enzyme activity $A_{Enz}$(measured) is used to determine a corrected first concentration $c1_{corr}$ of the fluorophore and/or of the analyte.

6. The method according to claim 5, wherein the determining of the corrected first concentration $c1_{corr}$ comprises an iterative algorithm.

7. The method according to any of the preceding claims, wherein the first measurement variable is one of absorption and fluorescence intensity.

8. The method according to claim 7, wherein the first measurement variable is absorption, and wherein the method further comprises measuring a third measurement variable which is characteristic for the quantity of the fluorophore, wherein the third measurement variable is fluorescence intensity, and wherein the first, the second and the third measurement variable are used in the evaluation algorithm.

9. The method according to any of the preceding claims, wherein the measurement of the fluorescence lifetime is used to determine a lifetime-related value of the fluorescence intensity.

10. The method according to claim 9, wherein the fluorescence intensity is measured for at least two different fluorescence lifetimes in order to determine in each case lifetime-related values of the fluorescence intensity.

11. The method according to claim 10, wherein the two lifetime-related values of the fluorescence intensity are related to one another.

12. The method according to any of the preceding claims, wherein the measured fluorescence lifetime is used in a compensation algorithm to compensate for measurement errors which are caused by interfering variables.

13. The method according to any of the preceding claims, wherein the fluorophore is NADH or NADPH or a derivative thereof, whose pyridine ring is not modified, in particular carbaNADH or carbaNADPH or a derivative thereof.

14. The method according to any of the preceding claims, wherein the enzyme is selected from the group consisting of glucose dehydrogenase (E.C.1.1.1.47), lactate dehydrogenase (E.C.1.1.1.27, 1.1.1.28), malate dehydrogenase (E.C.1.1.1.37), glycerine dehydrogenase (E.C.1.1.1.6), alcohol dehydrogenase (E.C.1.1.1.1), amino acid dehydrogenase, such as L-amino acid dehydrogenase (E.C.1.4.1.5), alpha-hydroxybutyrate dehydrogenase, sorbitol dehydrogenase, glucose oxidase (E.C.1.1.3.4), cholesterol oxidase (E.C.1.1.3.6), amino transferases, such as aspartate or alanine amino transferase, 5'-nucleotidase, creatin kinase, glucose 6-phosphate dehydrogenase (E.C.1.1.1.49), NAD dependent cholesterol dehydrogenase (E.C.1.1.1.62), and FAD dependent glucose dehydrogenase (E.C.1.1.99.10).

15. The method according to any of the preceding claims, wherein the analyte is glucose.

**Patentansprüche**

1. Verfahren zur Bewertung der Qualität eines Analyseelements, das sich zur Bestimmung der Konzentration eines Analyten in einer flüssigen Probe eignet, umfassend:

(a) Bereitstellen eines Analyseelements, in dem ein Reagentiensystem operativ integriert ist, wobei das Reagentiensystem wenigstens ein Enzym und wenigstens ein Coenzym enthält, wobei eine Umsetzung der Probe mit dem Reagentiensystem eine Komplexbildung wenigstens des Enzyms und des Coenzyms beinhaltet, und wobei eine chemisch modifizierte Form des Coenzyms ein Fluorophor ist;

(b) Umsetzen der Probe mit dem Reagentiensystem, um eine Änderung der Menge und/oder der Lumineszenzeigenschaften des Fluorophors hervorzurufen, wobei die Menge und/oder die Lumineszenzeigenschaften des Fluorophors in Beziehung zur Konzentration des Analyten stehen;

(c) gegebenenfalls Messen einer ersten Messvariablen;

(c') Messen wenigstens einer zweiten Messvariablen,

wobei die erste und die zweite Messvariable für die Menge des Fluorophors und/oder für den Grad der Komplexierung des Fluorophors charakteristisch sind und wobei es sich bei der zweiten Messvariablen um die Fluoreszenzlebensdauer des Fluorophors handelt;

(d) gegebenenfalls Bestimmen einer ersten Konzentration $c1_{initial}$ des Fluorophors und/oder des Analyten auf der Grundlage der ersten Messvariablen unter Verwendung einer vorgegebenen Enzymaktivität $A_{Enz}$(preset);

(e) Bestimmen einer zweiten Konzentration $c2_{ref}$ des Fluorophors und/oder des Analyten auf der Grundlage der zweiten Messvariablen unter Verwendung der vorgegebenen Enzymaktivität $A_{Enz}$(preset); und/oder Bestimmen der gemessenen Enzymaktivität $A_{Enz}$(measured) auf der Grundlage der zweiten Messvariablen unter Berücksichtigung entweder der ersten Konzentration $c1_{initial}$ aus Schritt (d) oder einer bekannten Konzentration $c1_{known}$ des Analyten in der Probe; und

(f) Bewerten der Qualität des Analyseelements unter Verwendung eines Bewertungsalgorithmus, wobei der Bewertungsalgorithmus Erhalten von Informationen bezüglich der gemessenen Enzymaktivität $A_{Enz}$(measured) umfasst, wobei die Informationen wenigstens teilweise aus der Messung der Fluoreszenzlebensdauer des Fluorophors gewonnen werden, und zwar entweder direkt durch Bestimmen der gemessenen Enzymaktivität $A_{Enz}$(measured) gemäß Schritt (e) oder indirekt durch Vergleichen der zweiten Konzentration $c2_{ref}$ mit der ersten Konzentration $c1_{initial}$.

2. Verfahren nach Anspruch 1, wobei der Bewertungsalgorithmus Bestimmen, ob die Enzymaktivität $A_{Enz}$(measured) unterhalb eines vorbestimmten Schwellenwerts T1 liegt, und/oder Bestimmen, ob der Unterschied zwischen der Enzymaktivität $A_{Enz}$(measured) und der vorgegebenen Enzymaktivität $A_{Enz}$(preset) oberhalb eines vorbestimmten Schwellenwerts T2 liegt, umfasst.

3. Verfahren nach Anspruch 1 oder 2, wobei der Bewertungsalgorithmus Bestimmen, ob der Unterschied zwischen der ersten Konzentration $c1_{initial}$ aus Schritt (d) und der zweiten Konzentration $c2_{ref}$ aus Schritt (e) oberhalb eines vorbestimmten Schwellenwerts T3 liegt, umfasst.

4. Verfahren nach Anspruch 2 oder 3, wobei das Analyseelement verworfen wird, falls die Enzymaktivität $A_{Enz}$ (measured) unterhalb des vorbestimmten Schwellenwerts T1 liegt und/oder falls der Unterschied zwischen der Enzymaktivität $A_{Enz}$(measured) und der vorgegebenen Enzymaktivität $A_{Enz}$(preset) oberhalb des vorbestimmten Schwellenwerts T2 liegt und/oder falls der Unterschied zwischen der ersten Konzentration $c1_{initial}$ aus Schritt (d) und der zweiten Konzentration $c2_{ref}$ aus Schritt (e) oberhalb des vorbestimmten Schwellenwerts T3 liegt.

5. Verfahren nach einem der vorhergehenden Ansprüche, wobei die Informationen über die Enzymaktivität $A_{Enz}$(measured) zum Bestimmen einer korrigierten ersten Konzentration $c1_{corr}$ des Fluorophors und/oder des Analyten verwendet werden.

6. Verfahren nach Anspruch 5, wobei das Bestimmen der korrigierten ersten Konzentration $c1_{corr}$ einen iterativen Algorithmus umfasst.

7. Verfahren nach einem der vorhergehenden Ansprüche, wobei es sich bei der ersten Messvariablen entweder um Absorption oder Fluoreszenzintensität handelt.

8. Verfahren nach Anspruch 7, wobei es sich bei der ersten Messvariablen um Absorption handelt und wobei das Verfahren ferner Messen einer dritten Messvariablen, die für die Menge des Fluorophors charakteristisch ist, umfasst, wobei es sich bei der dritten Messvariablen um Fluoreszenzintensität handelt und wobei die erste, die zweite und die dritte Messvariable im Bewertungsalgorithmus verwendet werden.

9. Verfahren nach einem der vorhergehenden Ansprüche, wobei die Messung der Fluoreszenzlebensdauer zum Bestimmen eines mit der Lebensdauer in Beziehung stehenden Werts der Fluoreszenzintensität verwendet wird.

10. Verfahren nach Anspruch 9, wobei die Fluoreszenzintensität für wenigstens zwei unterschiedliche Fluoreszenzlebensdauern gemessen wird, um jeweils mit der Lebensdauer in Beziehung stehende Werte der Fluoreszenzintensität zu bestimmen.

11. Verfahren nach Anspruch 10, wobei die beiden mit der Lebensdauer in Beziehung stehenden Werte der Fluoreszenzintensität in Beziehung zueinander stehen.

12. Verfahren nach einem der vorhergehenden Ansprüche, wobei die gemessene Fluoreszenzlebensdauer in einem Kompensationsalgorithmus zum Kompensieren von Messfehlern, die durch Störgrößen verursacht werden, verwendet wird.

13. Verfahren nach einem der vorhergehenden Ansprüche, wobei es sich bei dem Fluorophor um NADH oder NADPH oder ein Derivat davon, dessen Pyridinring nicht modifiziert ist, insbesondere carbaNADH oder carbaNADPH oder ein Derivat davon, handelt.

14. Verfahren nach einem der vorhergehenden Ansprüche, wobei das Enzym aus der aus Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), Aminosäure-Dehydrogenase, z.B. L-Aminosäure-Dehydrogenase (E.C.1.4.1.5), Alpha-Hydroxybutyrat-Dehydrogenase, Sorbitol-Dehydrogenase, Glucose-Oxidase (E.C.1.1.3.4), Cholesterin-Oxidase (E.C.1.1.3.6), Aminotransferasen, z.B. Aspartat- oder Alanin-Aminotransferase, 5'-Nukleotidase, Creatinkinase, Glucose-6-phosphat-Dehydrogenase (E.C.1.1.1.49), NAD-abhängiger Cholesterin-Dehydrogenase (E.C.1.1.1.62) und FAD-abhängiger Glucose-Dehydrogenase (E.C.1.1.99.10) bestehenden Gruppe ausgewählt ist.

15. Verfahren nach einem der vorhergehenden Ansprüche, wobei es sich bei dem Analyten um Glucose handelt.

**Revendications**

1. Procédé d'évaluation de la qualité d'un élément d'analyse qui est utile pour déterminer la concentration d'un analyte dans un échantillon liquide, comprenant :

   (a) la fourniture d'un élément d'analyse dans lequel un système de réactifs est opérationnellement intégré, dans lequel le système de réactifs contient au moins une enzyme et au moins une coenzyme, dans lequel une réaction de l'échantillon avec le système de réactifs inclut une formation de complexe d'au moins l'enzyme et la coenzyme, et

dans lequel une forme chimiquement modifiée de la coenzyme est un fluorophore ;

(b) la réaction de l'échantillon avec le système de réactifs afin de produire un changement de la quantité et/ou des propriétés de luminescence du fluorophore, dans lequel la quantité et/ou les propriétés de luminescence du fluorophore se rapportent à la concentration de l'analyte ;

(c) facultativement la mesure d'une première variable de mesure ;

(c') la mesure d'au moins une deuxième variable de mesure,

dans lequel la première et la deuxième variable de mesure sont caractéristiques de la quantité du fluorophore et/ou du degré de complexation du fluorophore, et dans lequel la deuxième variable de mesure est la durée de vie de fluorescence du fluorophore ;

(d) facultativement la détermination d'une première concentration $c1_{initial}$ du fluorophore et/ou de l'analyte sur la base de la première variable de mesure, en utilisant une activité enzymatique $A_{Enz}(preset)$ prédéfinie ;

(e) la détermination d'une deuxième concentration $c2_{ref}$ du fluorophore et/ou de l'analyte sur la base de la deuxième variable de mesure, en utilisant l'activité enzymatique $A_{Enz}(preset)$ prédéfinie ; et/ou la détermination de l'activité enzymatique $A_{Enz}(measured)$ mesurée sur la base de la deuxième variable de mesure et en prenant en compte soit la première concentration $c1_{initial}$ de l'étape (d), soit une concentration connue $c1_{known}$ de l'analyte dans l'échantillon ; et

(f) l'évaluation de la qualité de l'élément d'analyse en utilisant un algorithme d'évaluation, dans lequel l'algorithme d'évaluation comprend l'obtention d'informations se rapportant à l'activité enzymatique $A_{Enz}(measured)$ mesurée, dans lequel lesdites informations sont au moins partiellement dérivées de la mesure de la durée de vie de fluorescence du fluorophore, soit directement en déterminant l'activité enzymatique $A_{Enz}(measured)$ mesurée conformément à l'étape (e), soit indirectement en comparant la deuxième concentration $c2_{ref}$ à la première concentration $c1_{initial}$.

2. Procédé selon la revendication 1, dans lequel l'algorithme d'évaluation comprend la détermination si l'activité enzymatique $A_{Enz}(measured)$ est inférieure à un seuil T1 prédéterminé, et/ou la détermination si la différence entre l'activité enzymatique $A_{Enz}(measured)$ et l'activité enzymatique $A_{Enz}(pre-set)$ prédéfinie est supérieure à un seuil T2 prédéterminé.

3. Procédé selon la revendication 1 ou 2, dans lequel l'algorithme d'évaluation comprend la détermination si la différence entre la première concentration $c1_{initial}$ de l'étape (d) et la deuxième concentration $c2_{ref}$ de l'étape (e) est supérieure à un seuil T3 prédéterminé.

4. Procédé selon la revendication 2 ou 3, dans lequel l'élément d'analyse est rejeté si l'activité enzymatique $A_{Enz}(measured)$ est inférieure au seuil T1 prédéterminé, et/ou si la différence entre l'activité enzymatique $A_{Enz}(measured)$ et l'activité enzymatique $A_{Enz}(preset)$ prédéfinie est supérieure au seuil T2 prédéterminé, et/ou si la différence entre la première concentration $c1_{initial}$ de l'étape (d) et la deuxième concentration $c2_{ref}$ de l'étape (e) est supérieure au seuil T3 prédéterminé.

5. Procédé selon l'une quelconque des revendications précédentes, dans lequel les informations relatives à l'activité enzymatique $A_{Enz}(measured)$ sont utilisées pour déterminer une première concentration corrigée $c1_{corr}$ du fluorophore et/ou de l'analyte.

6. Procédé selon la revendication 5, dans lequel la détermination de la première concentration corrigée $c1_{corr}$ comprend un algorithme itératif.

7. Procédé selon l'une quelconque des revendications précédentes, dans lequel la première variable de mesure est une d'une absorption et d'une intensité de fluorescence.

8. Procédé selon la revendication 7, dans lequel la première variable de mesure est une absorption, et dans lequel le procédé comprend en outre la mesure d'une troisième variable de mesure qui est caractéristique de la quantité du fluorophore, dans lequel la troisième variable de mesure est une intensité de fluorescence, et dans lequel la première, la deuxième et la troisième variable de mesure sont utilisées dans l'algorithme d'évaluation.

9. Procédé selon l'une quelconque des revendications précédentes, dans lequel la mesure de la durée de vie de fluorescence est utilisée pour déterminer une valeur liée à la durée de vie de l'intensité de fluorescence.

10. Procédé selon la revendication 9, dans lequel l'intensité de fluorescence est mesurée pour au moins deux durées de vie de fluorescence différentes afin de déterminer dans chaque cas des valeurs liées à la durée de vie de l'intensité de fluorescence.

**11.** Procédé selon la revendication 10, dans lequel les deux valeurs liées à la durée de vie de l'intensité de fluorescence sont liées l'une à l'autre.

**12.** Procédé selon l'une quelconque des revendications précédentes, dans lequel la durée de vie de fluorescence mesurée est utilisée dans un algorithme de compensation pour compenser des erreurs de mesure qui sont causées par des variables interférentes.

**13.** Procédé selon l'une quelconque des revendications précédentes, dans lequel le fluorophore est NADH ou NADPH ou un dérivé de celui-ci, dont le cycle pyridine n'est pas modifié, en particulier carbaNADH ou carbaNADPH ou un dérivé de celui-ci.

**14.** Procédé selon l'une quelconque des revendications précédentes, dans lequel l'enzyme est choisie parmi le groupe consistant en la glucose déshydrogénase (E.C.1.1.1.47), la lactate déshydrogénase (E.C.1.1.1.27, 1.1.1.28), la malate déshydrogénase (E.C.1.1.1.37), la glycérine déshydrogénase (E.C.1.1.1.6), l'alcool déshydrogénase (E.C.1.1.1.1), une acide aminé déshydrogénase, telle que la L-acide aminé déshydrogénase (E.C.1.4.1.5), l'alpha-hydroxybutyrate déshydrogénase, la sorbitol déshydrogénase, la glucose oxydase (E.C.1.1.3.4), la cholestérol oxydase (E.C.1.1.3.6), les amino transférases, telles que l'aspartate ou l'alanine amino transférase, la 5'-nucléotidase, la créatine kinase, la glucose 6-phosphate déshydrogénase (E.C.1.1.1.49), la cholestérol déshydrogénase NAD-dépendante (E.C.1.1.1.62), et la glucose déshydrogénase FAD-dépendante (E.C.1.1.99.10).

**15.** Procédé selon l'une quelconque des revendications précédentes, dans lequel l'analyte est le glucose.

**Fig. 1**

Fig. 2

Fig. 3

Fig. 4

Fig. 5

**Fig. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0293732 A2
- US 20050214891 A1
- US 20060003397 A1
- WO 9400602 A
- EP 2022859 A
- US 5485530 A
- EP 0561653 A1
- WO 2007012494 A
- US 20070026476 A
- US 3802842 A
- US 4061468 A
- US 20060003397 A

**Non-patent literature cited in the description**

- **J. R. LAKOVICZ.** Principles of Fluorescence Spectroscopy. Springer Science and Business Media, 2006
- **T. G. SCOTT et al.** Emission Properties of NADH. Studies of Fluorescence Lifetimes ... *J. Am. Chem. Soc.,* 1970, 687-695
- *J. T. Slama, Biochemistry,* 1989, vol. 27, 183
- *Biochemistry,* 1989, vol. 28, 7688